# DEMANDE DE BREVET EUROPEEN

(11) **EP 1 350 472 A1**
(43) Date de publication de la demande: **08.10.2003**
(21) Numéro de dépôt: 03006537.9
(22) Date de dépôt: 24.03.2003
(51) Int. Cl.: A61B 17/15

(54) **Materiel permettant la resection d'un fémur en vue de la mise en place d'un implant fémoral de prothèse de genou**

(30) Priorité: 26.03.2002 FR 0203760
(71) Demandeur: Bergue, Bertrand, 69300 Clauire (FR)
(72) Inventeur: Bergue, Bertrand, 69300 Clauire (FR)

(57) **Abrégé**

Ce matériel (3) comprend :
- une embase formée par une pièce de base (11) comprenant des moyens de positionnement (12) par rapport à une tige centro-médullaire (10), et par une pièce de support (13) montée coulissante par rapport à cette pièce de base (11);
- des moyens (14, 15, 50) de prise d'appui contre les zones postérieures des condyles (1 a) et contre la zone antérieure du fémur (1);
- des moyens (16) de déplacement micrométrique des moyens d'appui antérieur (15) par rapport à la pièce de support (13), et des moyens (57) de repérage de la position de ces.moyens d'appui antérieur (15) par rapport à cette pièce de support (13) ; et
- des moyens (15, 16) de déplacement de la pièce de support (13) dans la direction antéro-postérieure du fémur (1) et vers la partie antérieure du fémur (1).

## Description

La présente invention concerne un matériel permettant la résection d'un fémur en vue de la mise en place sur ce fémur d'un implant fémoral de prothèse de genou.

Un implant fémoral de prothèse de genou présente, comme cela est bien connu, une forme recourbée destinée à envelopper l'extrémité distale d'un fémur. Par sa face extérieure, cet implant reproduit la trochlée et les condyles fémoraux tandis que sur sa face intérieure, cet implant présente généralement des facettes antérieures, distales et postérieures, pouvant être raccordées les unes aux autres par des facettes intermédiaires obliques, destinées à venir en contact avec le fémur réséqué.

Une même prothèse de genou comprend une série d'implants fémoraux de différentes tailles permettant au praticien de déterminer, en fonction des dimensions du fémur, l'implant le plus approprié au traitement de l'articulation concernée.

La mise en place d'un tel implant fémoral implique par conséquent de procéder à des coupes fémorales antérieure, distale et postérieure, et éventuellement intermédiaires, permettant la mise en place de l'implant sur le fémur.

Il existe des matériels permettant de mettre en place sur le fémur des broches qui servent au positionnement de blocs de coupe, ces blocs de coupe permettant le guidage des instruments de coupe. Il existe notamment un matériel comprenant une tige centro-médullaire destinée à être insérée dans le canal médullaire du fémur, une embase pouvant être positionnée sur cette tige au niveau de l'extrémité distale du fémur, des moyens de positionnement de cette embase par rapport à cette tige selon le valgus que présente l'articulation à traiter, et des moyens de guidage pour la réalisation de perçages de mise en place ultérieure de broches au niveau de la face distale du fémur.

Les matériels existants ont pour inconvénients de laisser subsister des imprécisions dans le positionnement desdites broches, pouvant conduire à des risques de défauts de positionnement de l'implant par rapport au fémur, en particulier au défaut de positionnement résultant de l'erreur bien connue dite "de plane oblique".

Par ailleurs, le praticien dispose de certains choix opératoires en ce qui concerne la mise en place de l'implant fémoral, en particulier
- conservation ou non du ligament croisé postérieur ;
- positionnement ou non de l'implant par rapport aux condyles postérieurs selon une angulation externe plus ou moins prononcée.

Selon que le ligament croisé postérieur est conservé ou non, le positionnement de l'élément fémoral est différent dans le plan sagittal : les zones postérieures des condyles prothétiques doivent reproduire le plus fidèlement possible les zones postérieures des condyles naturels lorsque ce ligament est conservé, tandis que l'on privilégiera la reproduction de la zone antérieure du fémur en cas de non-conservation de ce ligament. Lorsque la gamme d'implants fémoraux ne comprend pas un implant dont la taille correspond exactement au fémur traité, le praticien choisit préférentiellement l'implant de taille immédiatement inférieure et adapte la position de l'implant en conséquence.

Les matériels existants permettent de mesurer le fémur pour déterminer l'implant de taille adaptée mais ne permettent pas de définir de manière précise et rapide la position adéquate de l'implant par rapport au fémur selon que le ligament croisé postérieur est conservé ou non. Dans le cas de la mise en place d'un implant de taille immédiatement inférieure, la réalisation des coupes se fait plus ou moins au jugé et fait grandement appel à l'expérience du praticien.

S'agissant de l'angulation externe précitée, les matériels existants ne permettent pas de déterminer précisément et rapidement la position de l'implant, notamment en combinaison de la position sagittale différente de l'implant selon la conservation ou non du ligament croisé postérieur.

Les matériels existants peuvent de plus induire des erreurs de repérage du niveau de coupe antérieure, pouvant conduire soit à ce que le bord antérieur supérieur de l'implant fasse saillie au-dessus de la corticale adjacente, formant ainsi un redan indésirable entre ce bord de l'implant et cette corticale, soit à ce que ce même bord de l'implant se trouve en dessous de la corticale adjacente, formant ainsi, avec cette corticale, un gradin également indésirable.

La présente invention vise à remédier à l'ensemble de ces inconvénients.

Son objectif principal est de fournir un matériel permettant de déterminer le positionnement précis de l'implant fémoral en fonction des choix opératoires du praticien, notamment en fonction de la conservation ou non du ligament croisé postérieur et du positionnement ou non de l'implant selon une angulation externe par rapport aux condyles postérieurs.

Un autre objectif de l'invention est de fournir un matériel permettant de réduire le risque de défaut de niveau de coupe antérieure, et donc d'éliminer le redan ou le gradin susceptible d'exister entre le bord antérieur supérieur de l'implant et la corticale adjacente.

Un objectif supplémentaire de l'invention est de fournir un matériel de structure simple à fabriquer, et dont l'utilisation et la stérilisation sont relativement faciles.

Le matériel concerné comprend, de manière connue en soi, une tige centro-médullaire destinée à être insérée dans le canal médullaire du fémur, une embase pouvant être positionnée sur cette tige au niveau de l'extrémité distale du fémur, des moyens de positionnement de cette embase par rapport à cette tige selon le valgus que présente l'articulation à traiter, et des moyens de guidage pour la mise en place de broches au niveau de la face antérieure du fémur et la réalisation de perçages sur la face distale du fémur.

Selon l'invention,
- l'embase est formée par une pièce de base comprenant lesdits moyens de positionnement par rapport à la tige centro-médullaire, et par une pièce de support montée coulissante par rapport à cette pièce de base dans la direction antéro-postérieure du fémur, cette pièce de support comprenant lesdits moyens de guidage ;
- le matériel comprend des moyens de prise d'appui contre les zones postérieures des condyles, reliés à cette pièce de support, ces moyens étant dénommés ci-après "moyens d'appui postérieur" ;
- le matériel comprend des moyens de prise d'appui contre la zone antérieure du fémur, reliés à cette pièce de support, ces moyens étant dénommés ci-après "moyens d'appui antérieur" ;
- le matériel comprend des moyens de déplacement micrométrique des moyens d'appui antérieur par rapport à la pièce de support, et des moyens de repérage de la position de ces moyens d'appui antérieur par rapport à cette pièce de support ; et
- le matériel comprend des moyens de déplacement de la pièce de support dans la direction antéro-postérieure du fémur et vers la partie antérieure du fémur, entre une position de référence, dans laquelle les moyens d'appui postérieur sont en contact avec les zones postérieures des condyles, et une position de déplacement, dans laquelle la pièce de support est déplacée vers la zone antérieure du fémur, des moyens étant prévus pour autoriser ce déplacement de la pièce de support par rapport aux moyens d'appui postérieur,
lesdits moyens de déplacement micrométrique permettant d'amener les moyens d'appui antérieur en appui contre la zone antérieure du fémur, puis, au-delà de cette prise d'appui, de déplacer ladite pièce de support vers la partie antérieure du fémur, depuis ladite position de référence jusqu'à ladite position de déplacement.

En pratique, la tige centro-médullaire est insérée dans le fémur au travers d'un trou intercondylien distal puis la pièce de base est positionnée de manière adéquate par rapport à cette tige selon l'angle de valgus de l'articulation traitée. La pièce support est glissée sur la pièce de base, puis les moyens d'appui postérieur sont amenés au contact de la zone postérieure des condyles et les moyens d'appui antérieur sont amenés au contact de la zone antérieure du fémur. Les moyens de repérage sont alors utilisés pour déterminer, en fonction de la distance de ces moyens d'appui respectifs, la taille de l'implant fémoral qu'il convient d'implanter.

Si une taille d'implant correspond précisément à la mesure relevée, les broches sont mises en place au niveau antérieur puis les perçages antérieurs sont réalisés, à l'aide desdits moyens de guidage que comprend la pièce de support.

Si une taille d'implant ne correspond pas précisément à la mesure relevée, lesdits moyens de déplacement micrométrique sont actionnés pour amener les moyens d'appui antérieur dans une position qui correspond à la taille d'implant immédiatement inférieure, ce qui provoque le coulissement de la pièce de support dans le sens antérieur du fémur, vers ladite position de déplacement. La distance entre ladite position de référence et ladite position de déplacement permet alors d'évaluer le niveau de coupe antérieure ou postérieure, selon la conservation ou non du ligament croisé postérieur, qu'il convient d'opérer pour la mise en place dudit implant de taille immédiatement inférieure.

S'il a été décidé de conserver le ligament croisé postérieur, les moyens de déplacement micrométrique sont actionnés pour ramener ladite pièce de support dans ladite position de référence puis les broches sont mises en place et les perçages précités sont réalisés. Un bloc de coupe approprié sera utilisé ensuite pour opérer une coupe qui permettra à l'implant de reproduire la partie postérieure des condyles.

S'il a été décidé de ne pas conserver le ligament croisé postérieur, et que la taille mesurée du fémur se situe entre deux tailles d'implants, lesdits moyens de déplacement micrométrique sont actionnés pour placer ladite pièce de support dans une position correspondant à la plus petite de ces deux tailles d'implants. Les broches sont mises en place et les perçages précités sont réalisés alors que lesdits moyens d'appui antérieur sont dans cette position, et un bloc de coupe approprié sera utilisé ensuite pour opérer une coupe qui permettra de reproduire prioritairement la partie antérieure du fémur.

Le matériel selon l'invention permet ainsi de déterminer facilement et de manière très précise la position de l'implant par rapport au fémur selon que le ligament croisé postérieur est conservé ou non, et de positionner précisément les perçages dans l'un et l'autre cas. La décision de conservation ou de non-conservation du ligament croisé postérieur peut être prise en cours d'opération.

Avantageusement, le matériel comprend des moyens de mesure de la distance séparant ladite position de référence et ladite position de déplacement de ladite pièce de support.

Ces moyens de mesure permettent ainsi de déterminer précisément cette distance et donc les niveaux de coupe à opérer.

De préférence, lesdits moyens d'appui antérieur sont conformés de manière à ce que le point de contact de ces moyens avec la zone antérieure du fémur se déplace, lorsque lesdits moyens, de déplacement micrométrique sont actionnés, simultanément dans la direction antéro-postérieure et dans le sens proximo-distal, de manière à ce que ce point de contact, dans les différentes positions de ces moyens d'appui antérieur, suive une ligne inclinée correspondant à la ligne inclinée que définissent, dans un plan sagittal, les bords antérieurs supérieurs des différents implants d'une même gamme d'implants de différentes tailles lorsque ces implants sont superposés dans un plan sagittal avec mise en coïncidence de leurs zones condyliennes postérieures.

Ce point de contact vient ainsi, dans une position donnée des moyens d'appui antérieur, en appui contre l'os en un emplacement immédiatement adjacent à celui qu'occupera ledit bord de l'implant correspondant. Les erreurs précitées de repérage du niveau de coupe antérieure, pouvant conduire soit à des redans soit à des gradins indésirables sont ainsi éliminées.

De préférence, dans le cas d'un tel déplacement selon ladite ligne inclinée,
- lesdits moyens d'appui antérieur sont solidaires d'une pièce comprenant des moyens de coulissement et ladite pièce de support comprend des moyens de coulissement pouvant coopérer avec ceux de cette pièce pour permettre ledit déplacement selon ladite ligne inclinée, et
- ladite pièce de base comprend une paroi contre laquelle la pièce comprenant les moyens d'appui antérieur vient buter lors du déplacement de la pièce de support dans la direction antéro-postérieure du fémur, cette venue en butée permettant de réaliser ledit déplacement des moyens d'appui antérieur par rapport à ladite pièce de support selon ladite ligne inclinée.

Le déplacement de la pièce de support et des moyens d'appui antérieur est ainsi réalisé simultanément.

Selon une forme de réalisation préférée de l'invention, lesdits moyens de positionnement de la pièce de base par rapport à la tige centro-médullaire comprennent :
- une série de douilles cylindriques percées d'alésages permettant l'engagement à coulissement de chacune de ces douilles sur la tige centro-médullaire, l'axe de l'alésage de chaque douille formant, avec l'axe de la douille, un angle déterminé correspondant à l'angle de valgus que peut présenter une articulation du genou ;
- un canon solidaire de la pièce de base, destiné à recevoir à coulissement, de manière à ajustée, la douille adaptée au traitement de l'articulation concernée ;
- des moyens de calage angulaire de chaque douille par rapport audit canon.

La douille correspondant à l'angle de valgus relevé par radiographie est ainsi sélectionnée parmi l'ensemble des douilles disponibles et est mise en place dans ledit canon pour permettre de réaliser le positionnement adéquat de ladite pièce de base par rapport à la tige centro-médullaire.

Avantageusement, chaque douille et ledit canon comprennent des moyens d'indexation permettant le calage de chaque douille selon deux positions angulaires distantes de 180°.

Ainsi, la même série de douilles peut être utilisée pour le traitement d'une articulation gauche et d'une articulation droite.

De préférence, ledit canon comprend au moins une rainure latérale débouchant dans sa cavité, et chaque douille comprend au moins une rainure latérale venant en coïncidence de cette rainure lorsque la douille est engagée dans le canon, la pièce de support comprenant au moins un rebord venant s'engager à coulissement ajusté dans ces rainures. La pièce de support permet ainsi d'assurer le maintien de la douille dans le canon.

De préférence,
- ladite pièce de support comprend au moins une glissière aménagée en elle, et
- lesdits moyens d'appui postérieur sont formés par au moins une pièce en forme de fourche, dont les branches sont conformées pour être engagées à coulissement dans cette glissière et pour venir en appui contre les condyles du fémur.

Avantageusement, dans ce cas, le matériel comprend plusieurs pièces en forme de fourche, dont une présente des branches coplanaires et dont au moins une autre présente des branches formant un angle entre elles correspondant à l'angulation externe qu'un praticien peut souhaiter dans le positionnement d'un implant par rapport au fémur.

L'une ou l'autre des pièces en forme de fourche peut être mise en place dans ladite glissière selon que ladite angulation externe est voulue ou non et selon l'angle désiré pour cette angulation externe.

Lesdits moyens prévus pour autoriser le déplacement de la pièce de support par rapport aux moyens d'appui postérieur peuvent alors comprendre soit un dimensionnement de ladite glissière tel qu'il permet à chaque pièce en forme de fourche de s'incliner par rapport à la pièce de support pour venir dans ladite position précitée de déplacement antérieur, soit être constitués par une légère souplesse élastique des branches de chaque pièce en forme de fourche, permettant à ces branches de ployer, dans le même but.

Lesdits moyens d'appui antérieur peuvent quant à eux comprendre un doigt palpeur monté coulissant le long d'une branche oblique solidaire de la pièce de support ; lesdits moyens de déplacement micrométrique comprennent alors une vis dont la tête prend appui contre cette branche et dont le corps est engagé au travers d'un trou taraudé aménagé dans ledit doigt palpeur ; lesdits moyens de repérage peuvent alors comprendre une graduation imprimée sur cette branche et un repère imprimé sur ce doigt palpeur.

Pour sa bonne compréhension, l'invention est à nouveau décrite ci-dessous en référence au dessin schématique annexé représentant, à titre d'exemple non limitatif, deux formes de réalisation possibles du matériel qu'elle concerne.

La figure 1 en est une vue de côté selon une première forme de réalisation, alors qu'il est mis en place sur l'extrémité distale d'un fémur ;

la figure 2 en est une vue de face, dans cette même position ;

les figures 3 à 5 sont des vues respectivement de côté, de face et de dessus d'une pièce de base qu'il comprend selon cette première forme de réalisation, la figure 5 montrant également, vue de dessus, une douille faisant partie d'une série de douilles qu'il comprend aussi ;

la figure 6 est une vue de cette douille, en coupe selon l'axe de cette douille ;

les figures 7 à 9 sont des vues respectivement de côté, de face et de dessus d'une pièce de support qu'il comprend selon cette première forme de réalisation, les figures 7 et 9 montrant également, respectivement de côté et de dessus, en éclaté, un doigt palpeur et une vis qu'il comprend ;

la figure 10 est une vue partielle, de côté et à échelle agrandie, de cette pièce de support et d'une pièce en forme de fourche qu'il comprend ;

la figure 11 est une vue de dessus de cette pièce en forme de fourche ;

la figure 12 est une vue de côté d'un implant fémoral destiné à être mis en place sur le fémur ;

la figure 13 est une vue d'une série d'implants de différentes tailles, vus de côté, superposés les uns aux autres ;

la figure 14 est une vue en perspective, avant montage, de deux pièces qu'il comprend selon la première forme de réalisation ; et

la figure 15 est une vue de côté du matériel selon cette deuxième forme de réalisation, alors qu'il est mis en place sur l'extrémité distale d'un fémur.

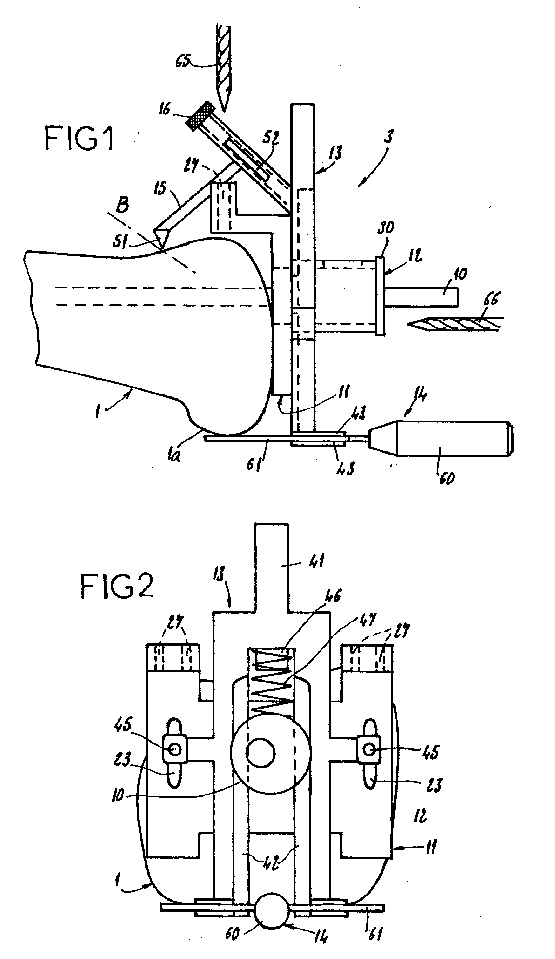

Les figures 1 et 2 représentent la portion distale d'un fémur 1 sur lequel doit être placé un implant fémoral de prothèse de genou.

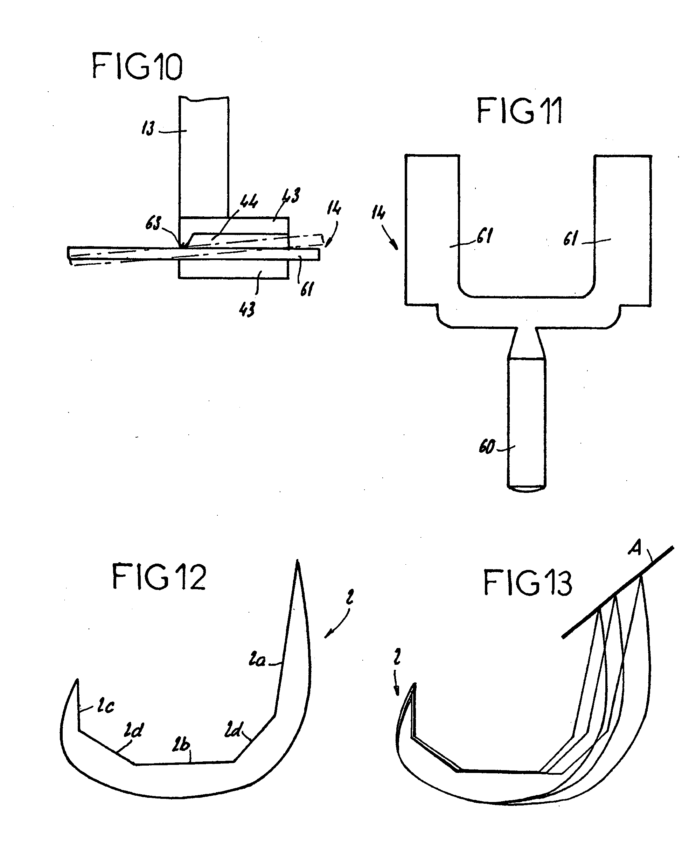

Comme le montre la figure 12, un tel implant 2 présente une forme recourbée destinée à envelopper l'extrémité distale du fémur 1 ; par sa face extérieure, cet implant 2 reproduit la trochlée et les condyles fémoraux et présente, sur sa face intérieure, des facettes antérieures 2a, distales 2b, postérieures 2c ainsi que des facettes intermédiaires obliques 2d, destinées à venir en contact avec le fémur 1 réséqué.

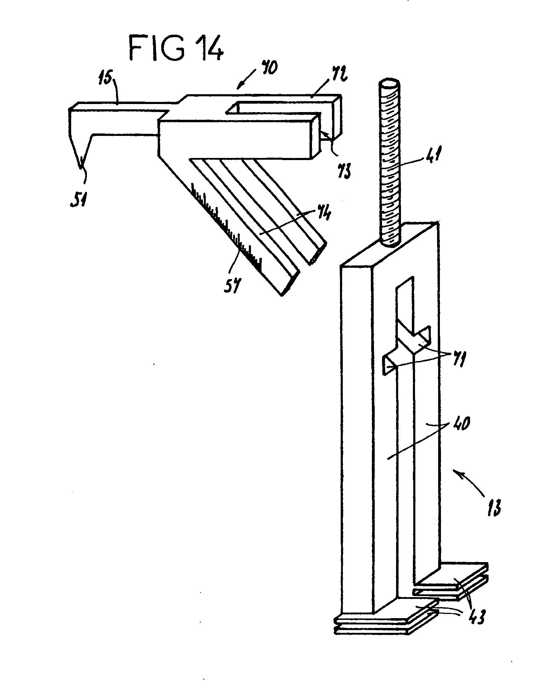

La figure 13 montre une superposition des différents implants 2 d'une même gamme d'implants de différentes tailles. Les bords antérieurs supérieurs de ces implants 2 définissent, dans le plan sagittal, une ligne inclinée A lorsque ces implants sont superposés avec mise en coïncidence de leurs zones condyliennes postérieures.

Pour la mise en place de l'implant 2 de taille appropriée, le fémur 1 doit être réséqué de manière adéquate, et le matériel 3 montré sur les figures 1 et 2 est utilisé à cet effet.

Ce matériel 3 comprend, selon ladite première forme de réalisation montrée aux figures 1 à 11, une tige centro-médullaire 10, une pièce de base 11, une série de douilles 12, une pièce de support 13, une série de pièces 14 en forme de fourche et un ensemble doigt palpeur 15 / vis 16 d'actionnement de ce doigt palpeur 15.

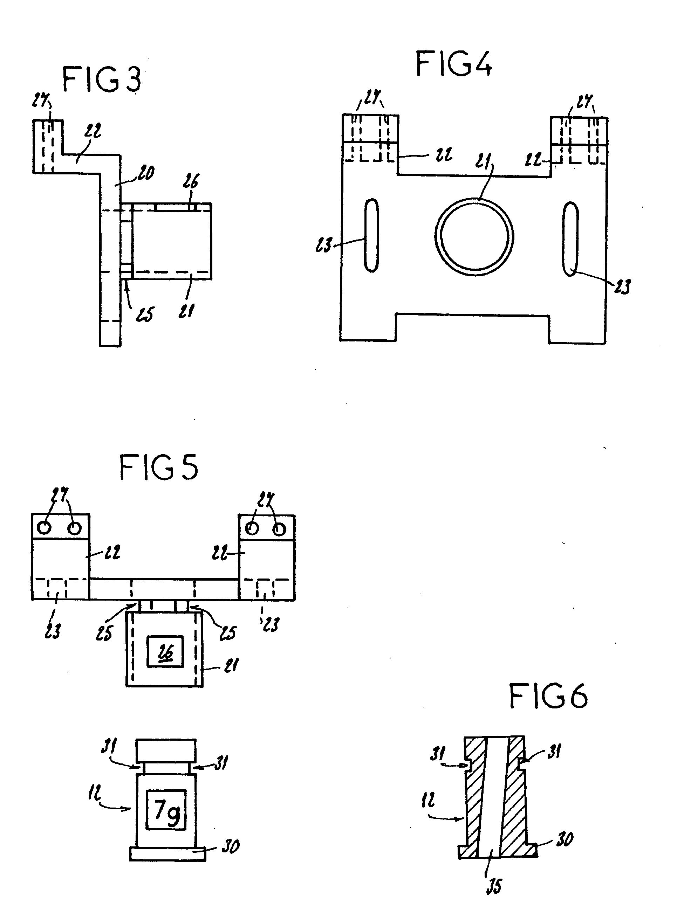

Comme cela apparaît sur les figures 3 à 5, la pièce de base 11 présente une partie 20 en forme de plaque, un canon central 21 faisant saillie de l'une des faces de cette partie 20, deux branches supérieures 22 faisant saillie de l'autre face de la partie 20, et deux lumières 23 aménagées au travers de la partie 20, de part et d'autre du canon 21.

Le canon 21 présente deux rainures rectilignes 25 diamétralement opposées, aménagées dans sa paroi, le long de la face de la partie 20, et mettant sa cavité en contact avec l'extérieur.

Le canon 21 comprend également une fenêtre supérieure 26. Les branches 22 comportent des canons 27 formant des guides de perçage.

Chaque douille 12 présente un diamètre tel qu'elle peut être engagée à coulissement, de manière ajustée, dans le canon 21. Elle comprend une collerette proximale 30 formant une butée limitant son engagement dans le canon 21 et deux rainures rectilignes latérales 31 diamétralement opposées, de dimensions correspondant à celles des rainures 25 du canon 21. Ces rainures 31 viennent en coïncidence des rainures 25 lorsque la collerette 30 est en butée contre le bord libre du canon 21 et peuvent recevoir à coulissement ajusté des rebords 42 de la pièce de support 13, décrits plus loin. L'engagement de ces rebords 42 dans ces rainures 25 et 31 permet d'assurer le maintien de la douille 12 dans le canon 21 et le calage angulaire de cette douille 12 par rapport à ce canon 21, selon deux positions angulaires distantes de 180°.

Comme le montre la figure 6, chaque douille 12 est percée d'un alésage 35 dont l'axe forme, avec l'axe de la douille 12, un angle déterminé correspondant à l'angle de valgus que peut présenter une articulation du genou.

Cet alésage 35 permet l'engagement à coulissement ajusté de chaque douille 12 sur la tige centro-médullaire 10, comme cela apparaît sur les figures 1 et 2.

En outre, sur deux faces diamétralement opposées, et au niveau de la zone de la douille 12 se trouvant en regard de la fenêtre 26 lorsque la collerette 30 est en butée contre le bord libre du canon 21, chaque douille 12 comporte une mention relative à l'angle que forme l'alésage 35 avec l'axe de la douille 12 et une indication "g" pour "gauche" ou "d" pour "droit" selon la direction de l'alésage 35 par rapport à l'axe de la douille 12.

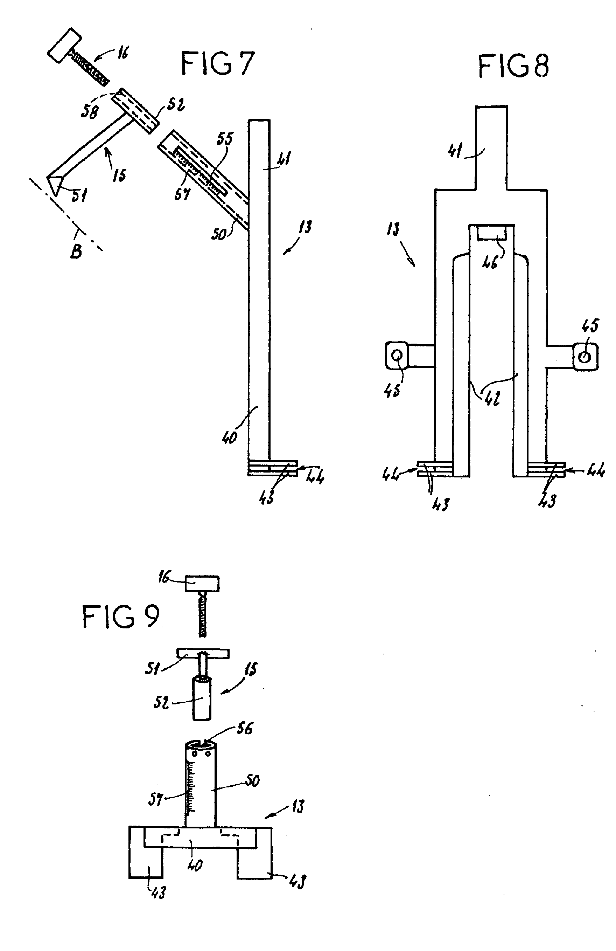

Comme le montrent les figures 7 à 10, la pièce de support 13 présente une forme de diapason inversé, c'est-à-dire comprend deux branches parallèles 40 délimitant un espace entre elles et une branche supérieure 41 de préhension.

Les branches 40 présentent des bords internes amincis, formant les rebords 42 précités d'engagement dans les rainures 25, 31, et comportent, à leur partie inférieure, des rebords 43 formant des glissières 44. Ces branches 40 comportent également des canons latéraux 45 formant des guides de perçage, orientés selon une direction perpendiculaire au plan de la pièce 13.

La pièce 13 comprend également un bossage 46 faisant saillie entre les branches 40, sur lequel est engagé avec frottements, et est ainsi retenu, un ressort hélicoïdal 47.

La pièce 13 comprend en outre une branche oblique supérieure 50 recevant à coulissement le doigt palpeur 15.

Comme cela se déduit des figures, l'inclinaison de cette branche 50 et l'inclinaison du doigt palpeur 15 lorsqu'il est monté sur cette branche 50 sont telles que la barrette de palpation 51 que comprend ce doigt 15 à une extrémité pour venir au contact de l'os suit une ligne B de même inclinaison que la ligne A lorsqu'un patin 52 que comprend le doigt 15 à son autre extrémité est déplacé par rapport à la branche 50.

Pour recevoir le doigt 15 à coulissement, la branche 50 comprend une cavité 55 de réception et de guidage du patin 52, débouchant sur l'extérieur, et une rainure inférieure communicant avec cette cavité 55, de guidage en coulissement de la base du doigt 15. La cavité 55 communique avec la face d'extrémité de la branche 50 par un alésage 56 destiné à recevoir la vis 16.

Le patin 52 comprend un repère et la branche 50 comprend une graduation 57 dont chaque marque correspond à une taille d'implant 2. Ce patin 52 comprend également un trou taraudé 58 destiné à recevoir la vis 16.

Pour le montage du doigt 15 sur la branche 50, le doigt 15 est engagé dans ladite rainure jusqu'à engagement total du patin 52 dans la cavité 55 puis la vis 16 est engagée dans l'alésage 56 et est vissée dans le trou 58 du patin 52. La vis 16 est ensuite immobilisée axialement par rapport à la branche 50, par exemple par engagement d'une broche transversale en U dans une gorge qu'elle comprend.

Chaque pièce 14 en forme de fourche comprend, ainsi que le montre la figure 11, un manche 60 et des branches 61. Le matériel 3 comprend plusieurs pièces 14, dont une présente des branches 61 coplanaires et chacune des autres présente des branches 61 formant un angle entre elles correspondant à une angulation externe qu'un praticien peut souhaiter dans le positionnement d'un implant 2 par rapport au fémur 1.

Il apparaît sur les figures 1, 2 et 10 que les glissières 44 formées par les rebords 43 sont destinées à recevoir les branches 61 de l'une ou l'autre des pièces 14. Comme le montre plus particulièrement la figure 10, les rebords 43 supérieurs présentent des nervures proximales 63 faisant saillie vers le bas, réduisant la hauteur des glissières 44, cette hauteur étant, en dehors de ces nervures 63, nettement supérieure à celle des branches 61 de chaque pièce 14. Il en résulte que chacune de ces pièces 14 peut prendre une position dans laquelle ses branches 61 sont sensiblement perpendiculaires à la direction longitudinale des branches 40 de la pièce 13, dite "position de référence", représentée en traits pleins sur la figure 10, et une position inclinée par rapport à cette même direction longitudinale, dite "position de déplacement", représentée en traits interrompus sur cette figure 10.

En pratique, l'angle de valgus de l'articulation à traiter est relevé par radiographie puis la douille 12 adaptée est mise en place dans le canon 21 et les rebords 42 de la pièce 13 sont engagés dans les rainures 25 et 31 jusqu'à appui du ressort 47 contre le canon 21. La tige centro-médullaire 10 est mise en place dans le fémur au travers d'un trou intercondylien percé à cet effet, puis l'ensemble pièce 11 / douille 12 / pièce 13 /doigt 15 et vis 16 montés sur la pièce 13 est mis en place sur cette tige 10 par engagement la douille 12 sur cette tige 10 et coulissement de cet ensemble jusqu'à venue de la face proximale de la partie 20 de la pièce 11 au contact des zones distales des condyles la. La pièce 13 est ensuite appuyée vers le bas de manière à comprimer le ressort 47, et la pièce 14 choisie par le praticien est engagée dans les glissières 44 ; l'appui sur la pièce 13 est alors relâché, ce qui amène les branches 61 de la pièce 14 au contact des zones postérieures des condyles la. La force du ressort 47 étant limitée, ces branches 61 restent dans ladite "position de référence".

Le matériel 3 ainsi positionné permet d'éviter toute erreur "de plane oblique", et des forets 65 de perçage de l'os peuvent être engagés et guidés au travers des canons 27.

La vis 16 est ensuite actionnée jusqu'à contact de la barrette 51 avec la zone antérieure du fémur 1. La graduation 57 permet de déterminer la taille de l'implant 2 correspondant.

Si une taille d'implant 2 correspond précisément à la mesure relevée, les canons 45 sont utilisés pour percer l'os au moyen de forets 66.

Si une taille d'implant 2 ne correspond pas précisément la mesure relevée, la vis 16 est actionnée pour amener le doigt 15 dans la position qui correspond à la taille d'implant 2 immédiatement inférieure, repérée grâce à la graduation 57 ; cet actionnement provoque le coulissement de la pièce 13 dans le sens antérieur du fémur 1 vers ladite "position de déplacement". La distance entre ladite "position de référence" et cette "position de déplacement" permet alors d'évaluer le niveau de coupe antérieure ou postérieure, selon la conservation ou non du ligament croisé postérieur, qu'il convient d'opérer pour la mise en place dudit implant 2 de taille immédiatement inférieure.

S'il a été décidé de conserver le ligament croisé postérieur, la vis 16 est actionnée pour ramener la pièce 13 dans ladite "position de référence" puis les perçages sont réalisés ; s'il a été décidé de ne pas conserver le ligament croisé postérieur, les perçages sont réalisés alors que la pièce 13 se trouve dans ladite position de déplacement.

Le matériel selon l'invention permet ainsi de déterminer facilement et de manière très précise la position de l'implant 2 par rapport au fémur 1 selon que le ligament croisé postérieur est conservé ou non, et de positionner précisément les perçages dans l'un et l'autre cas. La décision de conservation ou de non-conservation du ligament croisé postérieur peut être prise en cours d'opération.

L'inclinaison précitée de la branche 50 et du doigt 15 permet que la barrette 51 se déplace simultanément dans la, direction antéro-postérieure et dans le sens proximo-distal lorsque la vis 16 est actionnée, en suivant ladite ligne B. Cette barrette 51 vient ainsi, dans une position donnée du doigt 15, toujours en appui contre l'os en un emplacement immédiatement adjacent à celui qu'occupera le bord antérieur supérieur de l'implant 2 correspondant, ce qui permet d'éliminer les erreurs de repérage du niveau de coupe antérieure, pouvant conduire soit à des redans soit à des gradins indésirables entre ledit bord antérieur supérieur de l'implant 2 et la corticale adjacente.

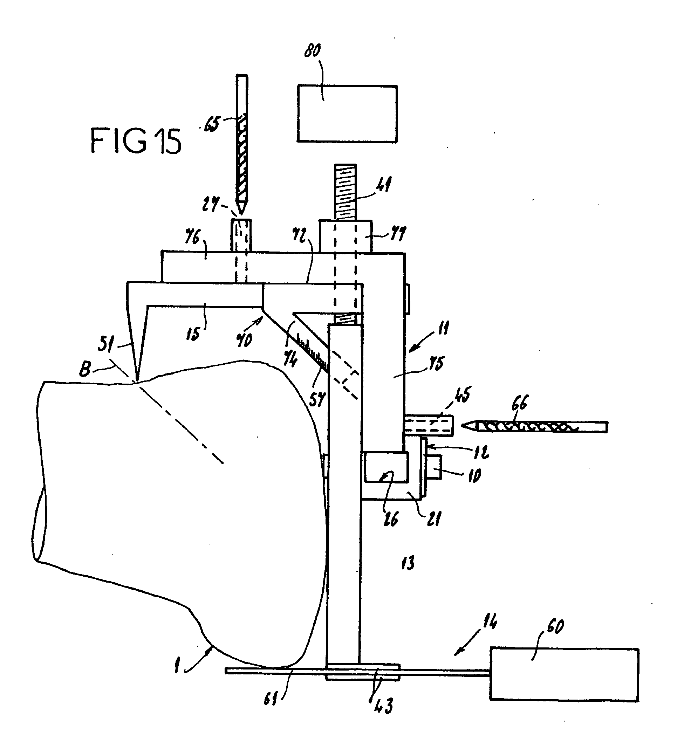

La figure 14 montre la pièce de support 13 et une pièce 70 formant le doigt palpeur 15 que comprend le matériel 3 selon ladite deuxième forme de réalisation, et la figure 15 montre ce matériel 3. Par simplification, les pièces, éléments, parties de pièces ou parties d'éléments déjà décrits, qui se retrouvent d'une forme de réalisation à l'autre, sont désignées par les mêmes références numériques et ne seront pas décrits dans le détail.

Comme le montre la figure 14, la pièce de support 13 comprend dans ce cas deux rainures inclinées 71 aménagées dans ses branches 40 à partir des faces internes de celles-ci, formant une glissière, et la branche 41 est filetée.

La pièce 70 incluant le doigt palpeur 15 comprend une branche supérieure 72 dans laquelle est aménagée une encoche 73, et deux branches inférieures inclinées 74. Ainsi que cela se comprend en référence à la figure 15, la branche 72 est destinée à être engagée autour de la branche 41 grâce à l'encoche 73, et les branches 74 sont destinées à être engagées et à coulisser dans la glissière formée par les rainures 71.

En référence à la figure 15, il apparaît que la pièce de base 11 présente une forme en 'V', dont une branche 75 est verticale et comporte le canon 21, et dont l'autre branche 76 est horizontale et est placée en partie supérieure ; cette branche horizontale 76 reçoit la face supérieure de la pièce 70 contre elle et comprend bossage supérieur 77, ce bossage 77 étant percé d'un alésage permettant l'engagement de la pièce de base 11 sur la tige 41.

Un écrou 80 est vissé sur cette dernière et vient porter, au cours de son vissage, contre la pièce de base 11.

En pratique, comme cela se comprend à partir de la figure 15, la pièce de base 11 est fixe puisqu'elle est liée à la tige centro-médullaire 10 par la douille 12 et le canon 21 ; le serrage de l'écrou 80 permet le déplacement de la pièce de support 13 et, simultanément, permet d'appuyer sur la pièce 70 afin de déplacer le doigt palpeur 15 selon la ligne B précitée.

Ainsi qu'il apparaît de ce qui précède, l'invention apporte une amélioration déterminante à la technique antérieure, en fournissant un matériel qui permet de déterminer le positionnement précis de l'implant 2 en fonction des choix opératoires du praticien, et de réduire le risque de défaut de niveau de coupe antérieure. Ce matériel reste d'une structure simple à fabriquer, et a une utilisation et une stérilisation relativement faciles.

Il va de soi que l'invention n'est pas limitée à la forme de réalisation décrite ci-dessus à titre d'exemple mais qu'elle en embrasse au contraire toutes les variantes de réalisation entrant dans le champ de protection défini par les revendications ci-annexées. Ainsi, les glissières 44 pourraient comprendre des ressorts; par exemple à lame, permettant de s'opposer normalement à l'inclinaison des branches 61 mais se déformant pour permettre cette inclinaison lorsque la pièce 13 est amenée dans ladite position de déplacement.

## Revendications

1. Matériel (3) permettant la résection d'un fémur (1) en vue de la mise en place sur ce fémur (1) d'un implant fémoral (2) de prothèse de genou, comprenant une tige centro-médullaire (10) destinée à être insérée dans le canal médullaire du fémur (1), une embase (11) pouvant être positionnée sur cette tige (10) au niveau de l'extrémité distale du fémur (1), des moyens de positionnement (12) de cette embase (11) par rapport à cette tige (10) selon le valgus que présente l'articulation à traiter, et des moyens de guidage (27, 45) pour la mise en place de broches au niveau de la face antérieure du fémur (1) et la réalisation de perçages sur la face distale du fémur (1) ;
matériel (3) **caractérisé :**
- **en ce que** l'embase est formée par une pièce de base (11) comprenant lesdits moyens de positionnement (12) par rapport à la tige centro-médullaire (10), et par une pièce de support (13) montée coulissante par rapport à cette pièce de base (11) dans la direction antéro-postérieure du fémur (1), cette pièce de support (13) comprenant lesdits moyens de guidage (27, 45) ;
- **en ce qu'**il comprend des moyens (14) de prise d'appui contre les zones postérieures des condyles (1a), reliés à cette pièce de support (13), ces moyens (14) étant dénommés ci-après "moyens d'appui postérieur" ;
- **en ce qu'**il comprend des moyens (15) de prise d'appui contre la zone antérieure du fémur (1), reliés à cette pièce de support (13), ces moyens (15) étant dénommés ci-après "moyens d'appui antérieur" ;
- **en ce qu'**il comprend des moyens (16) de déplacement micrométrique des moyens d'appui antérieur (15) par rapport à la pièce de support (13), et des moyens (57) de repérage de la position de ces moyens d'appui antérieur (15) par rapport à cette pièce de support (13) ; et
- **en ce qu'**il comprend des moyens (15, 16) de déplacement de la pièce de support (13) dans la direction antéro-postérieure du fémur (1) et vers la partie antérieure du fémur (1) entre une position de référence, dans laquelle les moyens d'appui postérieur (14) sont en contact avec les zones postérieures des condyles (1a), et une position de déplacement, dans laquelle la pièce de support (13) est déplacée vers la zone antérieure du fémur (1), des moyens (43, 44) étant prévus pour autoriser ce déplacement de la pièce de support (13) par rapport aux moyens d'appui postérieur (14),
lesdits moyens de déplacement micrométrique (16) permettant d'amener les moyens d'appui antérieur (15) en appui contre la zone antérieure du fémur (1), puis, au-delà de cette prise d'appui, de déplacer ladite pièce de support (13) vers la partie antérieure du fémur (1), depuis ladite position de référence jusqu'à ladite position de déplacement.

2. Matériel (3) selon la revendication 1, **caractérisé en ce qu'**il comprend des moyens de mesure de la distance séparant ladite position de référence et ladite position de déplacement de ladite pièce de support (13).

3. Matériel (3) selon la revendication 1 ou la revendication 2, **caractérisé en ce que** lesdits moyens d'appui antérieur (15) sont conformés de manière à ce que le point de contact de ces moyens (15) avec la zone antérieure du fémur (1) se déplace, lorsque lesdits moyens de déplacement micrométrique (16) sont actionnés, simultanément dans la direction antéro-postérieure et dans le sens proximo-distal, de manière à ce que ce point de contact, dans les différentes positions de ces moyens d'appui antérieur (15), suive une ligne inclinée (B) correspondant à la ligne inclinée (A) que définissent, dans un plan sagittal, les bords antérieurs supérieurs des différents implants (2) d'une même gamme d'implants (2) de différentes tailles lorsque ces implants (2) sont superposés dans un plan sagittal avec mise en coïncidence de leurs zones condyliennes postérieures.

4. Matériel (3) selon la revendication 3, **caractérisé en ce que** :
- lesdits moyens d'appui antérieur (15) sont solidaires d'une pièce (70) comprenant des moyens de coulissement (73, 74) et ladite pièce de support (13) comprend des moyens de coulissement (71) pouvant coopérer avec ceux de cette pièce (70) pour permettre ledit déplacement selon ladite ligne inclinée (B), et
- ladite pièce de base (11) comprend une paroi (76) contre laquelle la pièce (70) comprenant les moyens d'appui antérieur (15) vient buter lors du déplacement de la pièce de support (13) dans la direction antéro-postérieure du fémur (1), cette venue en butée permettant de réaliser ledit déplacement des moyens d'appui antérieur (15) par rapport à ladite pièce de support (13) selon ladite ligne inclinée (B).

5. Matériel (3) selon l'une des revendications 1 à 4, **caractérisé en ce que** lesdits moyens de positionnement de la pièce de base (11) par rapport à la tige centro-médullaire (10) comprennent :
- une série de douilles cylindriques (12) percées d'alésages (35) permettant l'engagement à coulissement de chacune de ces douilles (12) sur la tige centro-médullaire (10), l'axe de l'alésage (35) de chaque douille (12) formant, avec l'axe de la douille (12), un angle déterminé correspondant à l'angle de valgus que peut présenter une articulation du genou ;
- un canon (21) solidaire de la pièce de base (11), destiné à recevoir à coulissement, de manière à ajustée, la douille (12) adaptée au traitement de l'articulation concernée ;
- des moyens (25, 31) de calage angulaire de chaque douille (12) par rapport audit canon (21 ).

6. Matériel (3) selon la revendication 5, **caractérisé en ce que** chaque douille (12) et ledit canon (21) comprennent des moyens d'indexation (25, 31) permettant le calage de chaque douille (12) selon deux positions angulaires distantes de 180°.

7. Matériel (3) selon l'une des revendications 1 à 6, **caractérisé en ce que** :
- ladite pièce de support (13) comprend au moins une glissière (44) aménagée en elle, et
- lesdits moyens d'appui postérieur sont formés par au moins une pièce (14) en forme de fourche, dont les branches (61) sont conformées pour être engagées à coulissement dans cette glissière (44) et pour venir en appui contre les condyles (1a) du fémur (1).

8. Matériel (3) selon la revendication 7, **caractérisé en ce qu'**il comprend plusieurs pièces (14) en forme de fourche, dont une présente des branches (61) coplanaires et dont au moins une autre présente des branches (61) formant un angle entre elles correspondant à une angulation externe qu'un praticien peut souhaiter dans le positionnement d'un implant (2) par rapport au fémur (1).

9. Matériel (3) selon la revendication 7 ou la revendication 8, **caractérisé en ce que** lesdits moyens prévus pour autoriser le déplacement de la pièce de support (13) par rapport aux moyens d'appui postérieur (14) comprennent un dimensionnement de ladite glissière (44) tel qu'il permet à chaque pièce (14) en forme de fourche de s'incliner par rapport à la pièce de support (13) pour venir dans ladite position précitée de déplacement antérieur.

10. Matériel (3) selon la revendication 7 ou la revendication 8, **caractérisé en ce que** lesdits moyens prévus pour autoriser le déplacement de la pièce de support (13) par rapport aux moyens d'appui postérieur (14) sont constitués par une légère souplesse élastique des branches (61) de chaque pièce (14) en forme de fourche, permettant à ces branches (61) de ployer.
